# EUROPEAN PATENT APPLICATION

(11) **EP 4 411 628 A1**
(43) Date of publication of application: **07.08.2024**
(21) Application number: 23799239.1
(22) Date of filing: 28.04.2023
(51) Int. Cl.: G06T 3/40, G06T 17/00

(54) **SCANNING DATA PROCESSING METHOD AND APPARATUS, AND ELECTRONIC DEVICE AND MEDIUM**

(30) Priority: 02.05.2022 CN 202210477084
(71) Applicant: Shining 3D Tech Co., Ltd., Hangzhou, Zhejiang 311258 (CN)
(72) Inventor: ZHAO, Xiaobo, Hangzhou, Zhejiang 311258 (CN); CHEN, Xiaojun, Hangzhou, Zhejiang 311258 (CN); MA, Chao, Hangzhou, Zhejiang 311258 (CN); ZHANG, Wei, Hangzhou, Zhejiang 311258 (CN)
(74) Representative: Studio Torta S.p.A.
(86) International application number: PCT/CN2023/091808
(87) International publication number: WO 2023/213253

(57) **Abstract**

Embodiments of the present disclosure relate to a method and apparatus for processing scanning data, an electronic device, and a medium. The method includes: obtaining a plurality of scanned images including auxiliary feature points; and processing the plurality of scanned images to obtain three-dimensional coordinate points of the auxiliary feature points, and generating target scanning data based on the three-dimensional coordinate points of the auxiliary feature points and point cloud data. By adopting the above technical solution, the accuracy of positioning information of scan posts can be improved, so as to improve the efficiency and accuracy of processing scanning data in an intraoral scanning scenario.

## Description

### Cross Reference to Related Application

The present disclosure claims priority to Chinese Patent Application No.2022104770842, entitled "METHOD AND APPARATUS FOR PROCESSING SCANNING DATA, ELECTRONIC DEVICE , AND MEDIUM" filed with China National Intellectual Property Administration on May 2, 2022, which is incorporated herein by reference in its entirety.

### Technical Field

The present disclosure relates to the technical field of intraoral scanning, and in particular to a method and apparatus for processing scanning data, an electronic device, and a medium.

### Background

Typically, in scanning scenarios, relevant target positions are determined through scanning.

In the related art, due to the limitation of the scanning range, a multi-data stitching solution is usually used when data is scanned, ultimately resulting in not high overall accuracy of a model due to the existence of cumulative errors.

### Summary

### (I) Technical problem to be solved

The present disclosure aims to solve the technical problem that the overall accuracy of a model is not high in an existing scanning scenario.

### (II) Technical solution

In order to solve the above technical problem, embodiments of the present disclosure provide a method for processing scanning data, including:
obtaining a plurality of scanned images including auxiliary feature points;
processing the plurality of scanned images to obtain three-dimensional coordinate points of the auxiliary feature points; and
generating target scanning data based on the three-dimensional coordinate points of the auxiliary feature points and point cloud data.

An apparatus for processing scanning data is further provided in embodiments of the present disclosure, and includes:
an image obtaining component, configured to obtain a plurality of scanned images including auxiliary feature points;
an image processing component, configured to process the plurality of scanned images to obtain three-dimensional coordinate points of the auxiliary feature points; and
a generation component, configured to generate target scanning data based on the three-dimensional coordinate points of the auxiliary feature points and point cloud data.

An electronic device is further provided in embodiments of the present disclosure, and includes:
a memory;
a processor; and
a computer program.

The computer program is stored in the memory, and configured to be executed by the processor so as to implement the above method for processing scanning data.

A computer-readable storage medium is further provided in embodiments of the present disclosure, and stores a computer program. The computer program, when executed by a processor, implements the steps of the above method for processing scanning data.

### (III) Beneficial effects

Compared with the related art, the technical solution provided by the embodiments of the present disclosure has the following advantages:
According to the solution for processing scanning data provided by the embodiments of the present disclosure, the plurality of scanned images including the auxiliary feature points are obtained, the plurality of scanned images are processed to obtain the three-dimensional coordinate points of the auxiliary feature points, and the target scanning data is generated based on the three-dimensional coordinate points of the auxiliary feature points and the point cloud data. By adopting the above technical solution, the accuracy of positioning information of scan posts may be improved, so as to improve the efficiency and accuracy of processing scanning data in an intraoral scanning scenario.

It should be understood that the above general description and the following detailed description are only exemplary and explanatory, and cannot limit the present disclosure.

### Brief Description of Figures

Accompanying drawings herein are incorporated into a specification to form a part of the specification, illustrate embodiments conforming to the present disclosure, and are used for explaining the principle of the present disclosure together with the specification.

In order to describe technical solutions in embodiments of the present disclosure or in the related art more clearly, the accompanying drawings required to be used in descriptions of the embodiments or the related art will be briefly introduced below, and it is apparent that those of ordinary skill in the art can obtain other drawings according to these accompanying drawings without creative work.
FIG. 1 is a diagram of an application scenario of processing scanning data according to some embodiments of the present disclosure;
FIG. 2 is a schematic flowchart of a method for processing scanning data according to some embodiments of the present disclosure;
FIG. 3 is a schematic flowchart of another method for processing scanning data according to some embodiments of the present disclosure;
FIG. 4 is a schematic flowchart of another method for processing scanning data according to some embodiments of the present disclosure;
FIG. 5 is a schematic structural diagram of an apparatus for processing scanning data according to some embodiments of the present disclosure; and
FIG. 6 is a schematic structural diagram of an electronic device according to some embodiments of the present disclosure.

### Detailed Description

To make objectives, technical solutions, and advantages of embodiments of the present disclosure more clear, the technical solutions in the embodiments of the present disclosure are clearly and completely described as below, and it is apparent that the described embodiments are a part rather all of embodiments of the present disclosure. Based on the embodiments of the present disclosure, all other embodiments obtained by those of ordinary skill in the art without creative work shall fall within the scope of protection of the present disclosure.

During practical application, in a scenario of restoring missing teeth within an oral cavity, due to the limitation of the scanning range of an oral scanner, a multi-data stitching solution is usually used when intraoral data is scanned, ultimately resulting in not high overall accuracy of a model due to the existence of cumulative errors.

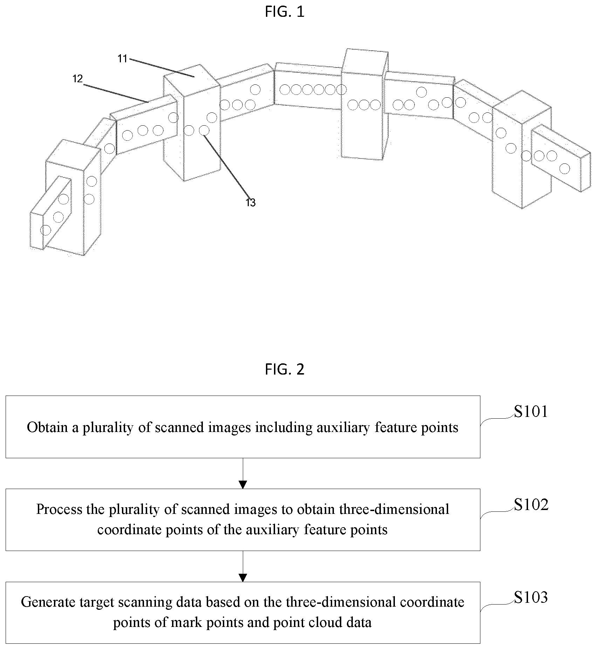

In response to the above problems, the present disclosure provides a method for processing scanning data, which may be applied to an application environment shown in FIG. 1. FIG. 1 is a diagram of an application scenario of processing scanning data according to some embodiments of the present disclosure. The application scenario includes: installing a plurality of intraoral scan posts in a target oral cavity. Each of the intraoral scan posts includes: a scan post component 11 and an auxiliary component 12 connected with the scan post component 11. The scan post component 11 and/or the auxiliary component 12 are/is provided with auxiliary feature points. The scan post component 11 is adaptive to an implant installed in the target oral cavity, and through adaptive installation of the scan post component 11 and the implant, the intraoral scan post is installed in the target oral cavity.

The auxiliary components 12 of any two of the plurality of intraoral scan posts are adaptive to each other, such that when the any two intraoral scan posts 10 are adjacently installed in the oral cavity, the auxiliary feature points on the two auxiliary components are continuously distributed. True value coordinate points of the auxiliary feature points may be obtained through a single-lens reflex photogrammetry system, a coordinate measuring machine, a high-precision industrial three-dimensional scanner, etc. In theory, three-dimensional coordinate points corresponding to an image obtained through scanning are in one-to-one correspondence with the true value coordinate points of the auxiliary feature points obtained in advance.

As an example scenario, a plurality of intraoral scan posts is installed in the target oral cavity. The intraoral scan post includes a scan post component used for being connected with the implant, and an auxiliary component connected with the scan post. The intraoral scan post has target features, and the target features are continuously distributed on the scan post and/or the auxiliary component, and are distributed non-unilaterally on the scan post and/or the auxiliary component.

The intraoral scanner scans the target oral cavity to obtain a plurality of frames of images, and transmits the images to a data processing component for data processing. The data processing component executes the following method:
A plurality of frames of images are obtained, initial three-dimensional data of the target oral cavity is acquired based on the plurality of frames of images, and the initial three-dimensional data includes an initial point set of the target oral cavity and three-dimensional coordinate measured values of the target features in the same coordinate system.

A preset model of the intraoral scan post is obtained, and includes three-dimensional coordinate true values of the target features and a real point set (three-dimensional coordinate true values of various points) of the intraoral scan post in the same coordinate system.

The initial point set of the target oral cavity and the real point set of the intraoral scan post are stitched based on a corresponding relationship between the three-dimensional coordinate measured values of the target features and the true values.

Positioning information of the intraoral scan post is determined based on the stitched real point set of the intraoral scan post, the positioning information of the intraoral scan post is positioning information of the implant, and dental prosthesis design is performed based on the positioning information, such that a designed and manufactured dental prosthesis can be adaptively installed with the implant.

By scanning the target oral cavity, a plurality of scanned images including auxiliary feature points are obtained; and the plurality of scanned images are processed to obtain three-dimensional coordinate points of the auxiliary feature points, and based on the three-dimensional coordinate points of the auxiliary feature points and point cloud data, target scanning data is generated. By adopting the above technical solution, the accuracy of positioning information of scan posts can be improved, so as to improve the efficiency and accuracy of processing scanning data in an intraoral scanning scenario. By adopting the above technical solution, the accuracy of positioning information of scan posts can be improved, so as to improve the scanning data processing efficiency and accuracy in an intraoral scanning scenario.

FIG. 2 is a schematic flowchart of a method for processing scanning data according to some embodiments of the present disclosure. The method can be executed by an apparatus for processing scanning data. The apparatus may be implemented by software and/or hardware, and may be typically integrated in an electronic device. As shown in FIG. 2, the method includes the following steps:
Step 101: A plurality of scanned images including auxiliary feature points are obtained.

The target oral cavity refers to an oral cavity needing dental implanting. Intraoral scanning needs to be performed so as to locate coordinates of specific points in the oral cavity. Scan posts are connected through auxiliary feature bodies for intraoral scanning.

In the embodiments of the present disclosure, the scan post includes the auxiliary feature body, which can have various shapes (e.g., features such as spherical, square, cuboid, and conical, or combinations of these features).

In the embodiments of the present disclosure, the scan post is a feature object including the auxiliary feature points. Each of the auxiliary feature points can uniquely identify one feature. That is, the scan post is provided with the auxiliary feature points, and each auxiliary feature point can uniquely identify a corresponding position feature on the scan post. For example, a target feature a and a target feature b are respectively set for a position 1 and a position 2 on the scan post, the target feature a can uniquely identify the position feature of the position 1 on the scan post, and the target feature b can uniquely identify the position feature of the position 2 on the scan post.

It is to be understood that different shapes, colors, two-dimensional codes, etc. on the scan post that can uniquely identify the corresponding position features on the scan post can be taken as the auxiliary feature points.

For example, convex or concave spherical and square shapes are arranged on the scan post and the auxiliary feature body thereof to serve as the auxiliary feature points, and for another example, different colors or different two-dimensional code patterns, different colors of circles and squares, etc. are printed on the scan post and the auxiliary feature body thereof. The specific settings are chosen based on application requirements.

In the embodiments of the present disclosure, after acquiring scan post data and auxiliary feature point data, a stitching algorithm is required to align the designed scan post data with the scan post data acquired in real time. Due to the non-rigid character of intraoral data during scanning through an intraoral scanner, the accuracy of one or more scan post data may deteriorate. Through the method in the embodiments of the present disclosure, the accuracy of aligning the designed scan post data with the real-time scan post data can be improved, thereby improving overall accuracy.

The target oral cavity can be scanned through a handheld intraoral scanner (monocular or binocular camera). In other words, the plurality of scanned images is obtained in a photography manner, for example, dozens of scanned images are collected per second, and cyclic acquisition can be performed.

The auxiliary feature point refers to a point arranged on a scan post body and/or the auxiliary feature body, which is set according to application scenarios.

In the embodiments of the present disclosure, there are various methods for scanning the target oral cavity including the scan posts to obtain a plurality of scanned images. In some embodiments of the present disclosure, a camera is controlled to rotate in a specific direction while scanning the target oral cavity at a certain frequency, thereby obtaining the plurality of scanned images.

After the target oral cavity is connected with the scan posts, the target oral cavity including the scan posts is scanned to obtain the plurality of scanned images.

Step 102: The plurality of scanned images are processed to obtain three-dimensional coordinate points of the auxiliary feature points.

The three-dimensional coordinate points of the auxiliary feature points refer to three-dimensional coordinate points corresponding to the auxiliary feature points in the target oral cavity.

In the embodiments of the present disclosure, there are various methods for processing the plurality of scanned images to obtain three-dimensional coordinate points of the auxiliary feature points. In some embodiments of the present disclosure, three-dimensional reconstruction is performed based on each scanned image to obtain a reconstructed image sequence. The reconstructed image sequence is calculated to obtain three-dimensional coordinate points of the auxiliary feature points and point cloud data.

In some other embodiments of the present disclosure, two-dimensional coordinate points of the auxiliary feature points in each scanned image are obtained and are subject to coordinate system transformation to obtain three-dimensional coordinate points of the auxiliary feature points of each scanned image. The three-dimensional coordinate points of the auxiliary feature points of each scanned image are stitched to obtain all three-dimensional coordinate points of the auxiliary feature points and point cloud data. The above two methods are only examples for processing the plurality of scanned images to obtain the three-dimensional coordinate points of the auxiliary feature points. This embodiment of the present disclosure does not limit a specific method for processing a plurality of scanned images to obtain three-dimensional coordinate points of auxiliary feature points.

In the embodiments of the present disclosure, after obtaining the plurality of scanned images, the plurality of scanned images can be processed to obtain the three-dimensional coordinate points of the auxiliary feature points.

Step 103: Target scanning data is generated based on the three-dimensional coordinate points of the auxiliary feature points and the point cloud data.

The three-dimensional coordinate points of the auxiliary feature points may be three-dimensional coordinate points obtained after optimization processing, which can more accurately reflect the three-dimensional coordinate points of the auxiliary feature points.

In the embodiments of the present disclosure, the step of generating target scanning data based on the three-dimensional coordinate points of the auxiliary feature points and the point cloud data may be understood as stitching the point cloud data of teeth, gums, etc. based on the three-dimensional coordinate points of the auxiliary feature points to obtain the target scanning data.

In the embodiments of the present disclosure, the step of generating target scanning data based on the three-dimensional coordinate points of the auxiliary feature points and the point cloud data includes: directly stitching the three-dimensional coordinate points of the auxiliary feature points and the point cloud data to obtain the target scanning data.

In the embodiments of the present disclosure, the step of generating target scanning data based on the three-dimensional coordinate points of the auxiliary feature points and the point cloud data includes: generating the target scanning data based on the three-dimensional coordinate points of the auxiliary feature points, the point cloud data, and standard data.

The standard data refers to scanning data capable of being stitched with the three-dimensional coordinate points of the auxiliary feature points. The standard scanning data can be acquired in advance through computer-aided designed scanning data, or through a single-lens reflex photogrammetry system, a coordinate measuring machine, and a high-precision industrial three-dimensional scanner. It should be understood that the standard scanning data can be pre-acquired and stored in a database so as to be directly acquired during processing, thereby improving the processing efficiency. The standard scanning data may also be acquired through real-time measurement according to a required scenario. The standard data may be standard scan post data in the intraoral scanning scenario.

The target scanning data replaces the real-time scanned scan post data after converting the computer-aided designed scan post data into the same coordinate system as the three-dimensional coordinate points of the auxiliary feature points.

In the embodiments of the present disclosure, there are various methods for generating target scanning data based on the three-dimensional coordinate points of the auxiliary feature points, the point cloud data, and the standard data. In some embodiments of the present disclosure, the auxiliary feature points have corresponding true value coordinate points, and the standard data is standard scan post data. Whether the three-dimensional coordinate points of the auxiliary feature points and the standard true value coordinate points of the auxiliary feature points are stitched is determined. If the three-dimensional coordinate points of the auxiliary feature points and the true value coordinate points of the auxiliary feature points are stitched, a position transformation matrix between the three-dimensional coordinate points of the auxiliary feature points and the true value coordinate points of the auxiliary feature points is obtained. Based on the position transformation matrix, the standard scan post data is transformed into the auxiliary feature point coordinate system and then is stitched with the three-dimensional coordinate points of the auxiliary feature points and the point cloud data to obtain the target scanning data.

In some other embodiments of the present disclosure, the standard data is standard scan post data. A plurality of planes is fitted based on the three-dimensional coordinate points of the auxiliary feature points. A target geometry is constructed based on the plurality of planes. At least three planes are obtained based on the standard scan post data, and a normal vector of each plane and intersection points of the at least three planes are obtained. Based on the normal vector of each plane and the intersection points of the at least three planes, a position transformation matrix is obtained. Based on the position transformation matrix, the standard scan post data is transformed into the auxiliary feature point coordinate system to replace the target geometry and be stitched with the point cloud data, such that the target scanning data is obtained.

In some other embodiments of the present disclosure, the auxiliary feature points have corresponding true value coordinate points, and the standard data is standard scan post data. The three-dimensional coordinate points of any one of the auxiliary feature points and the corresponding true value coordinate points of the auxiliary feature point are stitched to obtain three-dimensional coordinate points of the target auxiliary feature point in the same coordinate system. A position transformation matrix between the three-dimensional coordinate points of the target auxiliary feature point and the corresponding true value coordinate points of the auxiliary feature point is obtained. Based on the position transformation matrix, the standard scan post data is transformed into the auxiliary feature point coordinate system, and then is stitched with the three-dimensional coordinate points of the auxiliary feature point and the point cloud data to obtain the target scanning data.

The above three methods are only examples for generating target scanning data based on the three-dimensional coordinate points of the auxiliary feature points, the point cloud data, and the standard data. The embodiments of the present disclosure does not limit a specific method for generating target scanning data based on the three-dimensional coordinate points of the auxiliary feature points, the point cloud data, and the standard data.

After obtaining the three-dimensional coordinate points of the auxiliary feature points, the target scanning data can be generated based on the three-dimensional coordinate points of the auxiliary feature points, the point cloud data, and the standard data.

According to the solution for processing scanning data provided by the embodiments of the present disclosure, the plurality of scanned images is obtained. Each scanned image includes the auxiliary feature points. The plurality of scanned images is processed to obtain the three-dimensional coordinate points of the auxiliary feature points. The target scanning data is generated based on the three-dimensional coordinate points of the auxiliary feature points and the point cloud data. By adopting the above technical solution, the alignment accuracy between the designed scan post data and the real-time scan post data is improved, thereby improving the efficiency and accuracy of processing scanning data in the intraoral scanning scenario.

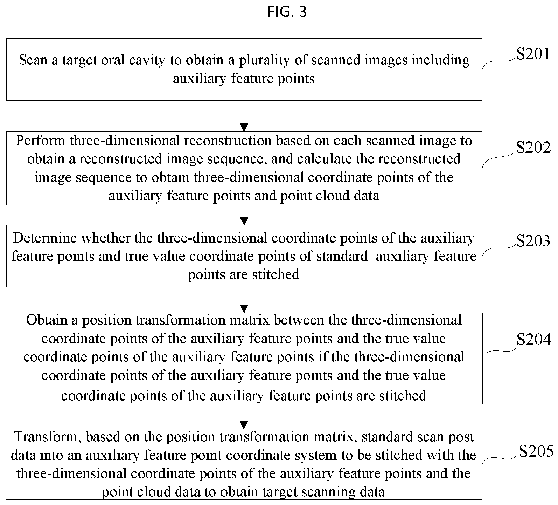

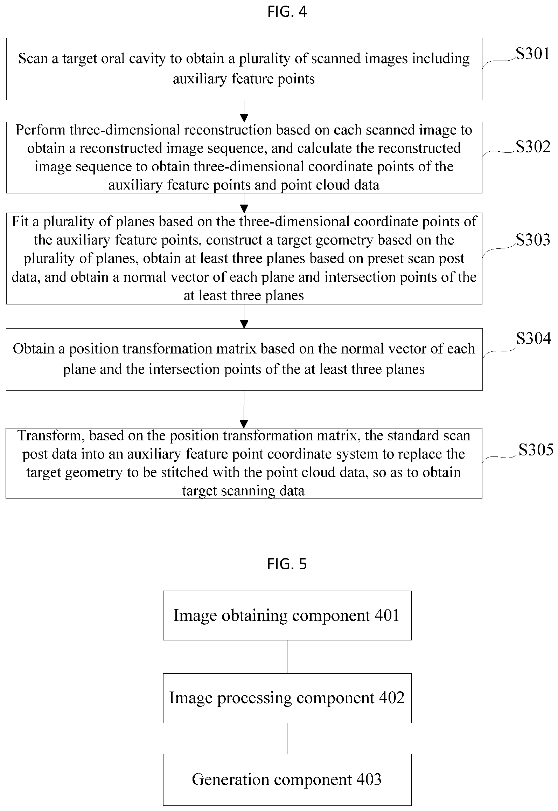

Methods for processing scanning data in different scenarios are respectively described in detail in conjunction with FIG. 3 and FIG. 4.

FIG. 3 is a schematic flowchart of another method for processing scanning data according to some embodiments of the present disclosure. In the embodiments, the above method for processing scanning data is further optimized based on the above embodiment. As shown in FIG. 3, the method includes the following steps:
Step 201: A target oral cavity is scanned to obtain a plurality of scanned images including auxiliary feature points.

Step 201 is the same as step 101. Please refer to the description of step 101 for details, which will not be repeated here.

Step 202: Three-dimensional reconstruction is performed based on each scanned image to obtain a reconstructed image sequence, and the reconstructed image sequence is calculated to obtain three-dimensional coordinate points of the auxiliary feature points and point cloud data.

The target oral cavity is scanned using the intraoral scanner to collect scanned images (reconstructed images and texture images) for three-dimensional reconstruction. The reconstructed image sequence is utilized for calculating three-dimensional data including teeth, gums, and the scan posts. The three-dimensional coordinate points of the auxiliary feature points are reconstructed using a texture image sequence. Simultaneous acquisition of the reconstructed images and the texture images may be considered as the three-dimensional data being in one-to-one correspondence with the three-dimensional coordinate points of the auxiliary feature points in the same coordinate system.

Step 203: Whether the three-dimensional coordinate points of the auxiliary feature points and the true value coordinate points of the standard auxiliary feature points are stitched is determined.

Step 204: If the three-dimensional coordinate points of the auxiliary feature points and the true value coordinate points of the auxiliary feature points are stitched, a position transformation matrix between the three-dimensional coordinate points of the auxiliary feature points and the true value coordinate points of the auxiliary feature points is obtained.

The auxiliary feature points are fabricated on the scan post body and the auxiliary feature body thereof. Relevant coordinates of the auxiliary feature points are defined as the true value coordinate points of the auxiliary feature points. In the scanning process, the obtained three-dimensional coordinate points of the auxiliary feature points and the true value coordinate points of the auxiliary feature points are matched. If a successful match is found, the designed scan post data corresponding to the current scan post can be replaced.

In the embodiments of the present disclosure, a first auxiliary feature point distance between the three-dimensional coordinate points of the auxiliary feature points and three-dimensional coordinate points of auxiliary feature points within a standard distance range is obtained, and the true value coordinate points of the auxiliary feature points matched with the three-dimensional coordinate points of the auxiliary feature points are obtained. A second auxiliary feature point distance between the true value coordinate points of the auxiliary feature points and true value coordinate points of the auxiliary feature points within the distance range is obtained. Based on the first auxiliary feature point distance and the second auxiliary feature point distance, whether the three-dimensional coordinate points of the auxiliary feature points and the true value coordinate points of the auxiliary feature points are stitched is determined. The distance range can be set according to application scenarios.

Step 205: The standard scan post data is transformed into an auxiliary feature point coordinate system based on the position transformation matrix and then is stitched with the three-dimensional coordinate points of the auxiliary feature points and the point cloud data to obtain target scanning data.

The collected three-dimensional coordinate points of the auxiliary feature points are stitched with the true value coordinate points of the standard auxiliary feature points. If stitching is successful, the position transformation matrix can be utilized for transforming the designed scan post data into the auxiliary feature point coordinate system to replace the scan post data acquired in real time.

According to the solution for processing scanning data provided by the embodiments of the present disclosure, the target oral cavity is scanned to obtain the plurality of scanned images. Each scanned image includes the auxiliary feature points. Three-dimensional reconstruction is performed based on each scanned image to obtain the reconstructed image sequence. The reconstructed image sequence is calculated to obtain the three-dimensional coordinate points of the auxiliary feature points and the point cloud data. Whether the three-dimensional coordinate points of the auxiliary feature points and the true value coordinate points of the standard auxiliary feature points are stitched is determined. If the three-dimensional coordinate points of the auxiliary feature points and the true value coordinate points of the auxiliary feature points are stitched, the position transformation matrix between the three-dimensional coordinate points of the auxiliary feature points and the true value coordinate points of the auxiliary feature points is obtained. Based on the position transformation matrix, the standard scan post data is transformed into the auxiliary feature point coordinate system and then is stitched with the three-dimensional coordinate points of the auxiliary feature points and the point cloud data to obtain the target scanning data. Therefore, the alignment accuracy between the designed scan post data and the real-time scan post data is improved, thereby improving the efficiency and accuracy of processing scanning data in the intraoral scanning scenario.

FIG. 4 is a schematic flowchart of another method for processing scanning data according to some embodiments of the present disclosure. In the embodiments, the above method for processing scanning data is further optimized based on the above embodiment. As shown in FIG. 4, the method includes the following steps:
Step 301: A target oral cavity is scanned to obtain a plurality of scanned images including auxiliary feature points.

Step 301 is the same as step 101. Please refer to the description of step 101 for details, which will not be repeated here.

Step 302: Three-dimensional reconstruction is performed based on each scanned image to obtain a reconstructed image sequence, and the reconstructed image sequence is calculated to obtain three-dimensional coordinate points of the auxiliary feature points and point cloud data.

The target oral cavity is scanned using the intraoral scanner to collect scanned images (reconstructed images and texture images) for three-dimensional reconstruction. The reconstructed image sequence is utilized for calculating point cloud data including teeth, gums, and the scan posts. The three-dimensional coordinate points of the auxiliary feature points are reconstructed using a texture image sequence. Simultaneous acquisition of the reconstructed images and the texture images may be considered as the three-dimensional data being in one-to-one correspondence with the three-dimensional coordinate points of the auxiliary feature points in the same coordinate system.

Step 303: A plurality of planes are fitted based on the three-dimensional coordinate points of the auxiliary feature points, a target geometry is constructed based on the plurality of planes, at least three planes are obtained based on standard scan post data, and a normal vector of each plane and intersection points of the at least three planes are obtained.

Step 304: A position transformation matrix is obtained based on the normal vector of each plane and the intersection points of the at least three planes.

Step 305: Based on the position transformation matrix, the standard scan post data is transformed into an auxiliary feature point coordinate system to replace the target geometry and be stitched with the point cloud data, such that target scanning data is obtained.

The position transformation matrix refers to a position transformation relationship that transforms the standard scan post data into the auxiliary feature point coordinate system.

Basic geometric features of the scan post are fitted through the plurality of auxiliary feature points. For example, the scan post is a cuboid, auxiliary feature points are distributed on 5 planes of the cuboid including 4 side surfaces and one top surface, and one cuboid can be respectively fitted through auxiliary feature point coordinates on each plane, thereby obtaining the target geometry.

Therefore, the auxiliary feature points obtained in the scanning process can fit the standard target geometry according to rules. The target geometry can directly serve as a position of the designed scan post data or be aligned with the designed scan post data once to determine the position of the designed scan post data.

According to the scanning data processing solution provided by the embodiments of the present disclosure, the target oral cavity is scanned to obtain the plurality of scanned images including the auxiliary feature points. Three-dimensional reconstruction is performed based on each scanned image to obtain the reconstructed image sequence. The reconstructed image sequence is calculated to obtain the three-dimensional coordinate points of the auxiliary feature points and the point cloud data. The plurality of planes is fitted based on the three-dimensional coordinate points of the auxiliary feature points. The target geometry is constructed based on the plurality of planes. The at least three planes are obtained based on the standard scan post data, and the normal vector of each plane and the intersection points of the at least three planes are obtained. Based on the normal vector of each plane and the intersection points of the at least three planes, the position transformation matrix is obtained. Based on the position transformation matrix, the standard scan post data is transformed into the auxiliary feature point coordinate system to replace the target geometry and be stitched with the point cloud data, such that the target scanning data is obtained. Therefore, the accuracy of positioning information of the scan posts can be improved, so as to improve the efficiency and accuracy of processing scanning data in the intraoral scanning scenario.

FIG. 5 is a schematic structural diagram of an apparatus for processing scanning data according to some embodiments of the present disclosure. The apparatus can be implemented by software and/or hardware, and can be typically integrated in an electronic device. As shown in FIG. 5, the apparatus includes:
an image obtaining component 401, configured to obtain a plurality of scanned images including auxiliary feature points;
an image processing component 402, configured to process the plurality of scanned images to obtain three-dimensional coordinate points of the auxiliary feature points; and
a generation component 403, configured to generate target scanning data based on the three-dimensional coordinate points of the auxiliary feature points and point cloud data.

The generation component 403 is configured to:
generate target scanning data based on the three-dimensional coordinate points of the auxiliary feature points, the point cloud data, and standard data.

The auxiliary feature points have corresponding true value coordinate points. The image processing component 402 is configured to:
perform three-dimensional reconstruction based on each of the scanned images to obtain a reconstructed image sequence; and
calculate the reconstructed image sequence to obtain three-dimensional coordinate points of the auxiliary feature points and point cloud data.

The generation component 403 includes:
a determination unit, configured to determine whether the three-dimensional coordinate points of the auxiliary feature points and true value coordinate points of the standard auxiliary feature points are stitched;
an obtaining unit, configured to obtain a position transformation matrix between the three-dimensional coordinate points of the auxiliary feature points and the true value coordinate points of the auxiliary feature points if the three-dimensional coordinate points of the auxiliary feature points and the true value coordinate points of the auxiliary feature points are stitched; and
a stitching unit, configured to transform, based on the position transformation matrix, the standard scan post data into an auxiliary feature point coordinate system to be stitched with the three-dimensional coordinate points of the auxiliary feature point and the point cloud data to obtain the target scanning data.

The determination unit is configured to:
determining whether the three-dimensional coordinate points of the auxiliary feature points and true value coordinate points of the standard auxiliary feature points are stitched, and this step includes:
obtaining a first auxiliary feature point distance between the three-dimensional coordinate points of the auxiliary feature points and three-dimensional coordinate points of auxiliary feature points within a standard distance range;
obtaining true value coordinate points of the auxiliary feature points matched with the three-dimensional coordinate points of the auxiliary feature points, and obtaining a second auxiliary feature point distance between the true value coordinate points of the auxiliary feature points and true value coordinate points of the auxiliary feature points within the distance range; and
determining whether the three-dimensional coordinate points of the auxiliary feature points and the true value coordinate points of the auxiliary feature points are stitched based on the first auxiliary feature point distance and the second auxiliary feature point distance.

The generation component 403 is configured to:
fit a plurality of planes based on the three-dimensional coordinate points of the auxiliary feature points, and construct a target geometry based on the plurality of planes;
obtain at least three planes based on the standard scan post data, and obtain a normal vector of each plane and intersection points of the at least three planes;
obtain a position transformation matrix based on the normal vector of each plane and the intersection points of the at least three planes; and
transform, based on the position transformation matrix, the standard scan post data into the auxiliary feature point coordinate system to replace the target geometry and be stitched with the point cloud data so as to obtain the target scanning data.

The auxiliary feature points have corresponding true value coordinate points. The generation component 403 is configured to:
stitch the three-dimensional coordinate points of any one of the auxiliary feature points and the corresponding true value coordinate points of the auxiliary feature point to obtain three-dimensional coordinate points of the target auxiliary feature point in the same coordinate system, obtain a position transformation matrix between the three-dimensional coordinate points of the target auxiliary feature point and the corresponding true value coordinate points of the auxiliary feature point, and transform, based on the position transformation matrix, the standard scan post data into the auxiliary feature point coordinate system to be stitched with the three-dimensional coordinate points of the auxiliary feature point and the point cloud data to obtain target scanning data.

The apparatus for processing scanning data provided by the embodiments of the present disclosure can execute the method for processing scanning data provided by any embodiments of the present disclosure, and has corresponding functional components and beneficial effects for executing the method.

Embodiments of the present disclosure further provide a computer program product including computer programs/instructions. The computer programs/instructions, when executed by a processor, implement the method for processing scanning data provided by any embodiments of the present disclosure.

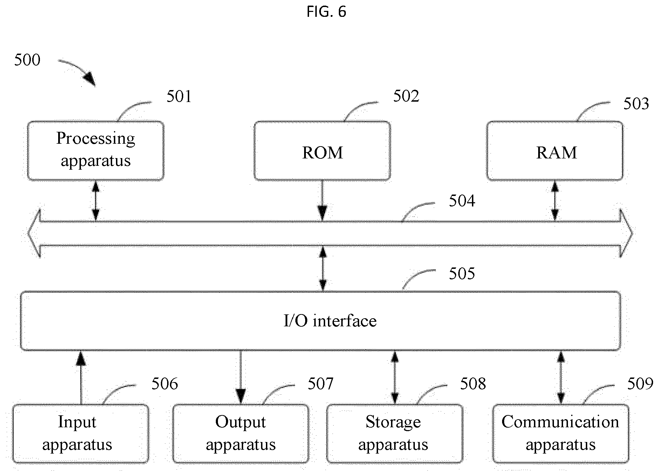

FIG. 6 is a schematic structural diagram of an electronic device according to some embodiments of the present disclosure. Referring to FIG. 6 below, FIG. 6 illustrates a schematic structural diagram of an electronic device 500 suitable for implementing some embodiments of the present disclosure. The electronic device 500 in the embodiments of the present disclosure may include, but is not limited to, mobile terminals such as a mobile phone, a notebook computer, a digital radio receiver, a personal digital assistant (PDA), a portable Android device (PAD), a portable media player (PMP), a vehicle-mounted terminal (e.g., a vehicle-mounted navigation terminal), and fixed terminals such as a digital TV and a desktop computer. The electronic device shown in FIG. 6 is merely an example, which should not impose any limitations on functions and application ranges of the embodiments of the present disclosure.

As shown in FIG. 6, the electronic device 500 may include a processing apparatus (e.g., a central processing unit and a graphics processing unit) 501, which may perform various appropriate actions and processing according to programs stored on a read only memory (ROM) 502 or loaded from a storage apparatus 508 into a random access memory (RAM) 503. The RAM 503 further stores various programs and data required for the operation of the electronic device 500. The processing apparatus 501, the ROM 502, and the RAM 503 are connected to one another through a bus 504. An input/output (I/O) interface 505 is also connected to the bus 504.

Typically, the following apparatuses may be connected to the I/O interface 505: an input apparatus 506, including, for example, a touchscreen, a touchpad, a keyboard, a mouse, a camera, a microphone, an accelerometer, and a gyroscope; an output apparatus 507, including, for example, a liquid crystal display (LCD), a speaker, and a vibrator; the storage apparatus 508, including, for example, a magnetic tape and a hard disk; and a communication apparatus 509. The communication apparatus 509 may allow the electronic device 500 to be in wireless or wired communication with other devices for data exchange. Although FIG. 6 illustrates the electronic device 500 with various apparatuses, it is to be understood that it is not necessary to implement or have all the shown apparatuses. Alternatively, more or fewer apparatuses may be implemented or provided.

The foregoing process described with reference to the flowchart according to the embodiments of the present disclosure may be implemented as a computer software program. For example, an embodiment of the present disclosure includes a computer program product including a computer program carried on a non-transitory computer-readable medium. The computer program includes program code for executing the method shown in the flowchart. In such an embodiment, the computer program may be downloaded and installed from a network by the communication apparatus 509, or installed from the storage apparatus 508, or installed from the ROM 502. The computer program, when executed by the processing apparatus 501, implements the above functions defined in the method for processing scanning data according to the embodiments of the present disclosure.

It should be noted that the computer-readable medium in the present disclosure may be a computer-readable signal medium, or a computer-readable storage medium, or any combination thereof. For example, the computer-readable storage medium may include, but is not limited to: electrical, magnetic, optical, electromagnetic, infrared, or semiconductor systems, apparatuses, or devices, or any combination thereof. More specific examples of the computer-readable storage medium may include, but are not limited to: an electrical connection with one or more wires, a portable computer disk, a hard disk, a random access memory (RAM), a read only memory (ROM), an erasable programmable read only memory (EPROM or a flash memory), fiber optics, a portable compact disc read only memory (CD-ROM), an optical storage device, a magnetic storage device, or any proper combination of the above. In the present disclosure, the computer-readable storage medium may be any tangible medium including or storing a program, and the program may be used by an instruction execution system, apparatus, or device, or used in conjunction with the instruction execution system, apparatus, or device. However, in the present disclosure, the computer-readable signal medium may include data signals propagated in a baseband or propagated as a part of a carrier wave, which carry computer-readable program code. The propagated data signals may have a plurality of forms, including, but not limited to, electromagnetic signals, optical signals, or any proper combination of the above. The computer-readable signal medium may be any computer-readable medium other than the computer-readable storage medium. The computer-readable signal medium may send, propagate, or transmit the program used by the instruction execution system, apparatus, or device, or used in conjunction with the instruction execution system, apparatus, or device. The program code included in the computer-readable medium may be transmitted by any proper medium including, but not limited to, a wire, an optical cable, radio frequency (RF), etc., or any proper combination of the above.

In some embodiments of the present disclosure, a client and a server can communicate using any currently known or future-developed network protocols such as a hyper text transfer protocol (HTTP), and may also be in communication connection with digital data in any form or medium (e.g., a communication network). For example, examples of the communication network include a local area network ("LAN"), a wide area network ("WAN"), Internet work (e.g., Internet), a peer-to-peer network (e.g., an ad hoc peer-to-peer network), and any currently known or future-developed networks.

The computer-readable medium may be included in the electronic device; and may separately exist without being assembled in the electronic device.

The computer-readable medium carries one or more programs. The one or more programs, when executed by the electronic device, enable the electronic device to: obtain a plurality of scanned images including auxiliary feature points, process the plurality of scanned images to obtain three-dimensional coordinate points of the auxiliary feature points, and generate target scanning data based on the three-dimensional coordinate points of the auxiliary feature points and point cloud data.

The computer program code for executing the operations of the present disclosure may be written in one or more programming languages or a combination thereof. The programming languages include, but are not limited to, object-oriented programming languages such as Java, Smalltalk, C++, as well as conventional procedural programming languages such as "C" or similar programming languages. The program code may be executed entirely or partially on a user computer, executed as a standalone software package, executed partially on the user computer and partially on a remote computer, or executed entirely on the remote computer or a server. In the case of involving the remote computer, the remote computer may be connected to the user computer via any type of network, including a local area network (LAN) or a wide area network (WAN), or may be connected to an external computer (e.g., utilizing an Internet service provider for Internet connectivity).

The flowcharts and block diagrams in the accompanying drawings illustrate system architectures, functions, and operations possibly implemented by the system, method and computer program product according to the various embodiments of the present disclosure. In this regard, each block in the flowcharts or block diagrams may represent a component, a program segment, or a portion of code, and the component, program segment, or portion of code includes one or more executable instructions for implementing specified logical functions. It should be noted that in some alternative implementations, functions marked in the blocks may also occur in an order different from that marked in the accompanying drawings. For example, two consecutively-shown blocks may be actually executed in parallel basically, but sometimes may also be executed in a reverse order, which depends on involved functions. It should be further noted that each block in the block diagrams and/or flowcharts as well as a combination of the blocks in the block diagrams and/or flowcharts may be implemented by using a dedicated hardware-based system that executes specified functions or operations, or using a combination of special hardware and computer instructions.

The units described in the embodiments of the present disclosure may be implemented through software or hardware. The name of the unit does not limit the unit in certain cases.

The functions described herein above may be at least partially executed by one or more hardware logic components. For example, exemplary hardware logic components that can be used include, but are not limited to, a field-programmable gate array (FPGA), an application specific integrated circuit (ASIC), an application specific standard part (ASSP), a system on chip (SOC), a complex programmable logic device (CPLD), etc.

In the context of the present disclosure, a machine-readable medium may be a tangible medium that may contain or store a program for use by the instruction execution system, apparatus, or device, or in conjunction with the instruction execution system, apparatus, or device. The machine-readable medium may be a machine-readable signal medium or a machine-readable storage medium. The machine-readable medium may include, but is not limited to: electronic, magnetic, optical, electromagnetic, infrared, or semiconductor systems, apparatuses, or devices, or any proper combination of the above. More specific examples of the machine-readable storage medium may include: an electrical connection based on one or more wires, a portable computer disk, a hard disk, a random access memory (RAM), a read only memory (ROM), an erasable programmable read only memory (EPROM or a flash memory), fiber optics, a portable compact disc read only memory (CD-ROM), an optical storage device, a magnetic storage device, or any proper combination of the above.

According to one or more embodiments of the present disclosure, the present disclosure provides an electronic device, including:
a processor; and
a memory configured to store executable instructions of the processor.

The processor is configured to read the executable instructions from the memory, and execute the instructions to implement any one of the methods for processing scanning data provided by the present disclosure.

According to one or more embodiments of the present disclosure, the present disclosure provides a computer-readable storage medium. The storage medium stores a computer program. The computer program is configured to execute any one of the methods for processing scanning data provided by the present disclosure.

In addition, embodiments of the present disclosure further provide an apparatus. The apparatus includes:
a processor; and
a memory configured to store executable instructions of the processor.

The processor is configured to:
obtain a plurality of scanned images including auxiliary feature points;
process the plurality of scanned images to obtain three-dimensional coordinate points of the auxiliary feature points and point cloud data; and
generate target scanning data based on the three-dimensional coordinate points of the auxiliary feature points and the point cloud data.

It should be noted that herein, relational terms such as "first" and "second" are used only to distinguish one entity or operation from another and do not necessarily require or imply any actual relationship or order between these entities or operations. In addition, terms "comprise", "include" or any other variations thereof are intended to cover non-exclusive inclusion, and therefore a process, a method, an article, or a device including a series of elements not only includes those elements but also includes other elements not clearly listed, or further includes inherent elements for the process, the method, the article, or the device. In the absence of further restrictions, an element specified by the phrase "including a..." does not exclude the existence of other identical elements in the process, method, article, or device that includes the element.

The above contents are merely implementations of the present disclosure, such that those skilled in the art can understand or implement the present disclosure. More modifications for these embodiments are apparent to those skilled in the art, and general principles defined herein may be implemented in other embodiments without departing from the spirit or scope of the present disclosure. Thus, the present disclosure will not be limited by these embodiments shown herein but is required to conform to a widest scope consistent with the principles and novel characteristics disclosed herein.

### Industrial applicability

The method for processing scanning data provided by the present disclosure can effectively calculate the scanning data, improves alignment accuracy between the designed scan post data and the real-time scan post data, can well consider influences on the scanning efficiency and accuracy in the intraoral scanning scenario, optimizes the scanning process, and has high industrial applicability.

## Claims

1. A method for processing scanning data, comprising:
obtaining a plurality of scanned images comprising auxiliary feature points;
processing the plurality of scanned images to obtain three-dimensional coordinate points of the auxiliary feature points and point cloud data; and
generating target scanning data based on the three-dimensional coordinate points of the auxiliary feature points and the point cloud data.

2. The method for processing scanning data as claimed in claim 1, wherein the generating the target scanning data based on the three-dimensional coordinate points of the auxiliary feature points and the point cloud data comprises:
generating the target scanning data based on the three-dimensional coordinate points of the auxiliary feature points, the point cloud data, and standard data.

3. The method for processing scanning data as claimed in claim 1, wherein the processing the plurality of scanned images to obtain the three-dimensional coordinate points of the auxiliary feature points and the point cloud data comprises:
performing three-dimensional reconstruction based on each of the scanned images to obtain a reconstructed image sequence; and
calculating the reconstructed image sequence to obtain the three-dimensional coordinate points of the auxiliary feature points and the point cloud data.

4. The method for processing scanning data as claimed in claim 2, wherein the auxiliary feature points have corresponding true value coordinate points, and the standard data is standard scan post data; and the generating the target scanning data based on the three-dimensional coordinate points of the auxiliary feature points, the point cloud data, and the standard data comprises:
determining whether the three-dimensional coordinate points of the auxiliary feature points and true value coordinate points of standard auxiliary feature points are stitched;
obtaining a position transformation matrix between the three-dimensional coordinate points of the auxiliary feature points and the true value coordinate points of the auxiliary feature points in response to that the three-dimensional coordinate points of the auxiliary feature points and the true value coordinate points of the auxiliary feature points are stitched; and
transforming, based on the position transformation matrix, the standard scan post data into an auxiliary feature point coordinate system to be stitched with the three-dimensional coordinate points of the auxiliary feature points and the point cloud data to obtain the target scanning data.

5. The method for processing scanning data as claimed in claim 2, wherein the standard data is standard scan post data, and the generating the target scanning data based on the three-dimensional coordinate points of the auxiliary feature points, the point cloud data, and the standard data comprises:
fitting a plurality of planes based on the three-dimensional coordinate points of the auxiliary feature points, and constructing a target geometry based on the plurality of planes;
obtaining at least three planes based on the standard scan post data, and obtaining a normal vector of each plane and intersection points of the at least three planes;
obtaining a position transformation matrix based on the normal vector of each plane and the intersection points of the at least three planes; and
transforming, based on the position transformation matrix, the standard scan post data into an auxiliary feature point coordinate system to replace the target geometry and being stitched with the point cloud data so as to obtain the target scanning data.

6. The method for processing scanning data as claimed in claim 2, wherein the auxiliary feature points have corresponding true value coordinate points, and the standard data is standard scan post data; and the generating the target scanning data based on the three-dimensional coordinate points of the auxiliary feature points, the point cloud data, and the standard data comprises:
stitching the three-dimensional coordinate points of any one of the auxiliary feature points and the corresponding true value coordinate points of the auxiliary feature points to obtain three-dimensional coordinate points of target auxiliary feature point in a same coordinate system;
obtaining a position transformation matrix between the three-dimensional coordinate points of the target auxiliary feature point and the corresponding true value coordinate points of the auxiliary feature points; and
transforming, based on the position transformation matrix, the standard scan post data into an auxiliary feature point coordinate system to be stitched with the three-dimensional coordinate points of the auxiliary feature points and the point cloud data to obtain the target scanning data.

7. An apparatus for processing scanning data, comprising:
an image obtaining component, configured to obtain a plurality of scanned images comprising auxiliary feature points;
an image processing component, configured to process the plurality of scanned images to obtain three-dimensional coordinate points of the auxiliary feature points; and
a generation component, configured to generate target scanning data based on the three-dimensional coordinate points of the auxiliary feature points and point cloud data.

8. The apparatus for processing scanning data as claimed in claim 7, wherein the generation component is configured to:
perform three-dimensional reconstruction based on each of the scanned images to obtain a reconstructed image sequence; and
calculate the reconstructed image sequence to obtain the three-dimensional coordinate points of the auxiliary feature points.

9. An electronic device, comprising:
a processor; and
a memory configured to store executable instructions of the processor,
the processor is configured to read the executable instructions from the memory, and execute the instructions to implement the method for processing scanning data as claimed in any one of claims 1 to 6.

10. A computer-readable storage medium, wherein the computer-readable storage medium stores a computer program, and the computer program is configured to execute the method for processing scanning data as claimed in any one of claims 1 to 6.
